# EUROPEAN PATENT SPECIFICATION

(11) **EP 3 124 053 B1**
(45) Date of publication and mention of the grant of the patent: **06.11.2024**
(21) Application number: 16180631.0
(22) Date of filing: 21.07.2016
(51) Int. Cl.: A61L 2/00, A61L 2/10, A61M 1/36

(54) **IRRADIATION DEVICE FOR BIOLOGICAL FLUIDS**
BESTRAHLUNGSVORRICHTUNG FÜR BIOLOGISCHE FLÜSSIGKEITEN
DISPOSITIF D'IRRADIATION DE FLUIDES BIOLOGIQUES

(30) Priority: 31.07.2015 US 201514815287
(43) Date of publication of application: 01.02.2017
(73) Proprietor: Fenwal, Inc., Lake Zurich, IL 60047 (US)
(72) Inventor: MANZELLA, Salvatore, Lake Zurich, IL 60047 (US); PIEPER, Gregory G., Lake Zurich, IL 60047 (US); CRAWLEY, Brett T., Lake Zurich, IL 60047 (US)
(74) Representative: Maikowski & Ninnemann Patentanwälte Partnerschaft mbB

(56) References cited:
- EP-A1- 1 079 868
- EP-B1- 1 079 868
- WO-A1-2011/031167
- WO-A1-2011/045243
- WO-A1-2017/019408
- WO-A1-96/40857
- WO-A2-2004/033081
- WO-A2-2007/027419
- DE-A1- 19 500 802
- US-A1- 2002 015 662
- US-A1- 2003 219 354
- US-A1- 2007 080 081
- US-A1- 2007 242 348
- US-B1- 6 800 432

## Description

This patent relates to devices, methods and systems for processing and treating biological fluids, such as blood and blood components. More particularly, the patent relates to devices, methods and systems involving irradiation of biological fluids, such as blood and blood components, in a container disposed in a treatment chamber.

An irradiation device is particularly useful in the treatment of biological fluids. As used herein, biological fluid refers to any fluid that is found in or that may be introduced into the body including, but not limited to, blood and blood products. As used herein, "blood product" refers to whole blood or a component of whole blood such as red blood cells, white blood cells, platelets, plasma or a combination of one or more of such components that have been separated from whole blood.

For example, an irradiation device may be used in the treatment of a blood product that has been combined with a photochemical agent for activation when subjected to light. Such photochemical agents may be used, for example, in the inactivation of viruses, bacteria and other contaminants (collectively referred to herein as "pathogens"). Photochemical agents are also used in the treatment of mononuclear cells, such as white blood cells. In pathogen inactivation applications, the activated agent inactivates pathogens that may be present in a blood product. In the treatment of mononuclear cells, the agent targets the mononuclear cells themselves as part of a treatment of a disease or a side effect of a mononuclear cell therapy. For example, WO 96/40857 A1 describes the inactivation of pathogens in blood products by photoactivation.

Typically, the biological fluid to be treated is introduced into a fluid treatment chamber within the irradiation device in flexible, plastic, sterilizable, translucent, biologically compatible containers. The containers may be integrally connected to other containers and plastic tubing useful in the processing of the biological fluid both before and after the treatment provided by the irradiation device.

One such irradiation device is described in U.S. Patent No. 7,433,030. The device includes a fluid carrying drawer with a central cavity to allow for placement of a container-carrying tray. The device includes upper and lower light drawers, each of which is separated from the fluid carrying drawer by a glass plate. The glass plates are substantially translucent to light of the wavelengths used for the treatment of biological fluid. In a specific embodiment, the glass plates are translucent to ultraviolet light within the range of 320-400 nm used for photopheresis, but not translucent to unwanted light, specifically light of a wavelength of less than 320 nm. For example, WO2004/70033081A1 discloses an irradiation device that includes upper and lower glass plates that separate the fluid carrying drawer from the light drawers. The glass plates preferably filter unwanted light. WO 99/59645 A1 refers to an apparatus for inactivating contaminants in biological fluid wherein the biological fluid is contacted with a light source providing high intensity light.

Biological fluids, and, more specifically, blood and suspensions of blood components, are temperature sensitive, and care needs to be taken to ensure that during treatment, in which light energy is being absorbed, it is not subjected to temperatures higher than about 41°C, and more preferably about 37°C, to limit hemolysis and cell death. To this end, the irradiation device in the above-referenced patent includes a blower for temperature control of the treatment chamber, and temperature sensors for monitoring and measuring the temperature within the fluid treatment chamber. If the temperature in the fluid treatment chamber falls outside of a predetermined range, an alert is sent to the operator that the temperature is approaching or has exceeded its limit, so that appropriate action may be taken, namely terminating the procedure and marking the container of biological fluid as "unusable."

By way of the present disclosure, an irradiation device is provided by which the temperature of the fluid treatment chamber of the device is moderated, so as to reduce the likelihood that the temperature of the biological fluid being treated is outside of a safe range.

Insofar as the term embodiment or aspect or alternative is used in the following, or features are presented as being optional, this should be interpreted in such a way that the only protection sought is that of the invention claimed and defined in the appended claims.

In a first aspect, a device for irradiation of a biological product/substance comprising a base; a tray having a first and second side associated with the base and configured to receive a container holding the biological product/substance to be irradiated; a first array of radiation-emitting bulbs mounted in the device adjacent the first side of the tray; a second array of radiation-emitting bulbs mounted in device adjacent a second side of the tray; a first filter mounted in the device between the tray and the first array of radiation-emitting bulbs for blocking and/or reflecting radiation emitted by the first array of a selected wavelength; and a second filter mounted in the device between the tray and the second array of radiation-emitting bulbs for blocking and/or reflecting radiation emitted by the second array of the selected wavelength; each of the first and second filters comprising a first surface facing the tray and a second surface facing away from the tray and toward its associated array of radiation-emitting bulbs, the second surface of each filter having a coating thereon for blocking and/or reflecting radiation of the selected wavelength.

Each of the first and second filters blocks and/or reflects infrared (IR) radiation, while permitting ultraviolet (UV) radiation (and, more specifically, UVA radiation) to pass therethrough.

In a third aspect, each of the first and second filters comprises a substrate with a coating thereon for blocking and/or reflecting radiation of the selected wavelength.

The substrate comprises glass and the coating comprises silver.

In a fifth aspect, the coating has a thickness of from 0.5 µm to 1.0 µm, and/or the substrate preferably has a thickness of 3.3 mm ⁺/- 1.3 mm.

In a sixth aspect, the device further comprises a fan or blower mounted within the base, and the base includes vents to permit air circulation and exchange around the tray.

In a seventh aspect, the speed of the fan is controlled to vary the output of UV radiation from the bulbs. Preferably, a UV sensor is positioned within the device in proximity to the radiation-emitting bulbs to measure the UVA output of the lamps, a signal indicative of which is transmitted to a controller, and the speed of the fan is increased or decreased by the controller to adjust the temperature of the lamps to obtain the desired UVA output. For example, the device further comprises a sensor for measuring the radiant output of the radiation-emitting bulbs and a controller configured to adjust the speed of the fan based on a signal received from the sensor to vary the radiant output of the radiation-emitting bulbs

In an eighth aspect, a method for irradiating a mononuclear cell product is provided comprising: placing a collection container suitable for irradiation into an irradiation chamber defined by an exterior surface having a coating thereon that blocks and/or reflects non-UVA light, the irradiation chamber being positioned between first and second light sources; introducing a suspension comprising mononuclear cells into the collection container; activating the first and second light sources to introduce UVA light into the irradiation chamber to expose the collection container to UVA light; and preventing non-UVA light from entering into the collection chamber.

In a ninth aspect, a device (system) for irradiating a biological product/substance comprises a container for holding the biological product/substance to be irradiated and an irradiation device. The irradiation device comprises a base; an openable cover; a tray associated with the base configured to receive the container of the biological product/substance to be irradiated; a first array of radiation-emitting bulbs mounted in the base beneath the tray; a second array of radiation-emitting bulbs mounted in the cover; a first filter mounted in the base between the tray and the first array of radiation-emitting bulbs for blocking and/or reflecting radiation emitted by the first array of a selected wavelength; and a second filter mounted in the cover between the tray and the second array of radiation-emitting bulbs for blocking and/or reflecting radiation emitted by the second array of the selected wavelength; each of the first and second filters comprising a first surface facing the tray and a second surface facing away from the tray and toward its associated array of radiation-emitting bulbs, the second surface of each filter having a coating thereon for blocking and/or reflecting radiation of the selected wavelength.
Fig. 1 is a perspective view of an irradiation device for biological fluids in accordance with the present disclosure.
Fig. 2 is a perspective view, in cross section, of the irradiation device of Fig. 1.
Fig. 3 is a fragmentary cross sectional view of the irradiation device of Fig. 1, enlarged to show detail.
Fig. 4 is a perspective view, in cross section, of a tray for supporting a biological fluid container that forms a part of the irradiation device.
Fig 5 is a schematic view of a microprocessor-based control system for adjusting the speed of a fan associated with the irradiation device.

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

As described herein, the irradiation device is a stand-alone device that may also be used in conjunction with a cell separator as part of a system. According to such a system, the cell separator would be configured to direct a biological fluid into a biological fluid container, and the irradiation device would include a fluid treatment chamber configured to receive the biological fluid container.

The cell separator may be an Amicus^{®} Separator made and sold by Fenwal, Inc., of Lake Zurich, Illinois, a subsidiary of Fresenius Kabi USA, LLC. Mononuclear cell collections using a device such as the Amicus^{®} are described in greater detail in U.S. Patent No. 6,027,657.

The container may be part of a fluid circuit (also referred to as a processing set) that includes a network of tubing and pre-connected containers for establishing flow communication with the patient and for processing and collecting fluids and blood and blood components.

An exemplary fluid circuit is disclosed in US 2013/0197419. As disclosed therein, the fluid circuit includes a separation chamber defined by the walls of a flexible processing container, as well as a container for supplying anticoagulant, a waste container for collecting waste from one or more steps in the process for treating and washing mononuclear cells, a container for holding saline or other wash or resuspension medium, a container for collecting plasma, a container for collecting the mononuclear cells from the operation discussed above and, optionally, a container for holding a photoactivation agent.

The mononuclear cell collection container may also serve as the illumination or irradiation container, and is preferably pre-attached to the disposable set. The fluid circuit includes an inlet line, an anticoagulant (AC) line for delivering AC from the AC container, an RBC line for conveying red blood cells from the separation chamber to a waste container, a platelet-poor plasma (PPP) line for conveying PPP to plasma container, and a line for conveying mononuclear cells between the separation chamber and the collection/illumination container. The blood processing set also includes one or more venipuncture needle(s) for accessing the circulatory system of the patient. The fluid circuit may include both an inlet needle and a return needle. Alternatively, a single needle can serve as both the inlet and return needle.

The mononuclear cell collection container is suitable for irradiation by light of a selected wavelength. By "suitable for irradiation", it is meant that the walls of the container are sufficiently translucent to light of the selected wavelength. In treatments using UVA light, for example, container walls made of ethylene vinyl acetate (EVA) are suitable. Accordingly, the container in which the mononuclear cells are collected may serve both as the collection container and the irradiation container. The collection/irradiation container may be placed inside the irradiation device by the operator or, more preferably, may be placed inside the irradiation chamber of irradiation device at the beginning of a procedure including the cell separator and prior to whole blood withdrawal. In any event, the mononuclear cell collection container preferably remains integrally connected to the remainder of the fluid circuit during the entire procedure, thereby maintaining the closed or functionally closed condition of the fluid circuit. Fluid flow through fluid circuit is preferably driven, controlled and adjusted by a microprocessor-based controller in cooperation with the valves, pumps, weight scales and sensors of the separation device and fluid circuit, the details of which are described in U.S. Patent No. 6,027,657, referenced above.

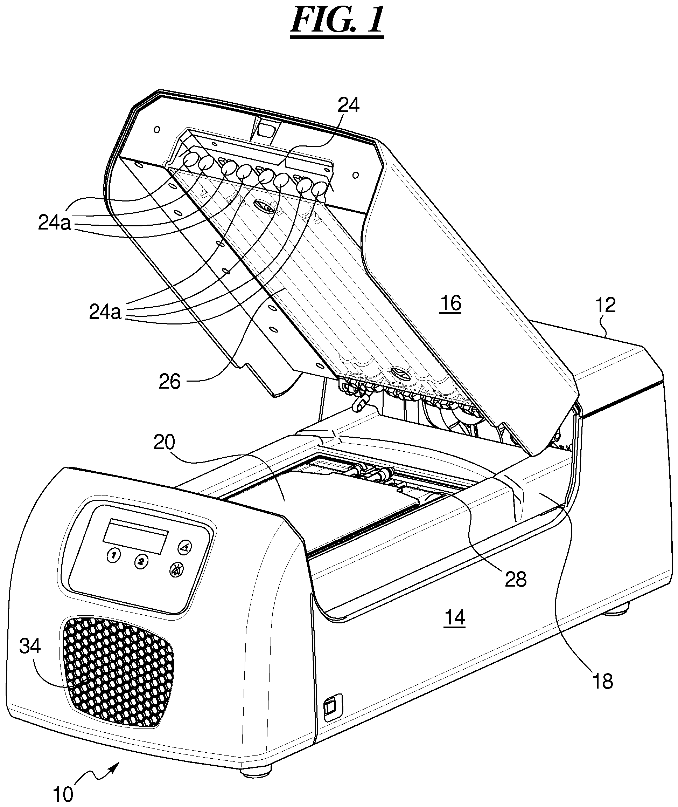

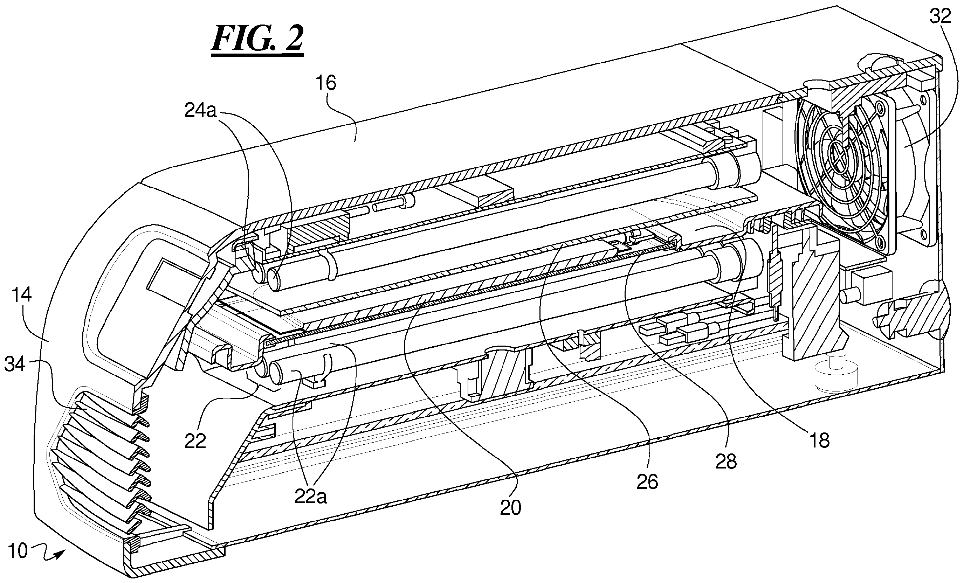

Turning to Figs. 1-4, an irradiation device, generally designated 10, is seen that comprises a housing 12 including a base 14 and a cover or closure 16. As illustrated, the cover is secured to the base 14 by a hinge so that the cover 16 can be pivoted about the hinge to provide access to the interior of the housing 12. The interior of the housing defines a fluid treatment chamber that includes a tray 18 configured to receive and support a biological fluid container 20.

The tray 18 has opposed first and second sides, one facing the base 14 and the other facing the cover 16. The tray 18 may be made, in part, of a polymeric material, with certain sections of the tray being made of another material, such as glass.

A light source is disposed on the interior of the device adjacent each of the first and second sides of the tray. As illustrated, the light source includes a first array 22, including a plurality of light sources 22a, mounted in the base on the first side of the tray, and a second array 24, including a plurality of light sources 24a, mounted in the cover 16 on the second side of the tray 18. According to the present disclosure, the light sources 22a, 24a provide electromagnetic radiation in the ultraviolet portion of the spectrum.

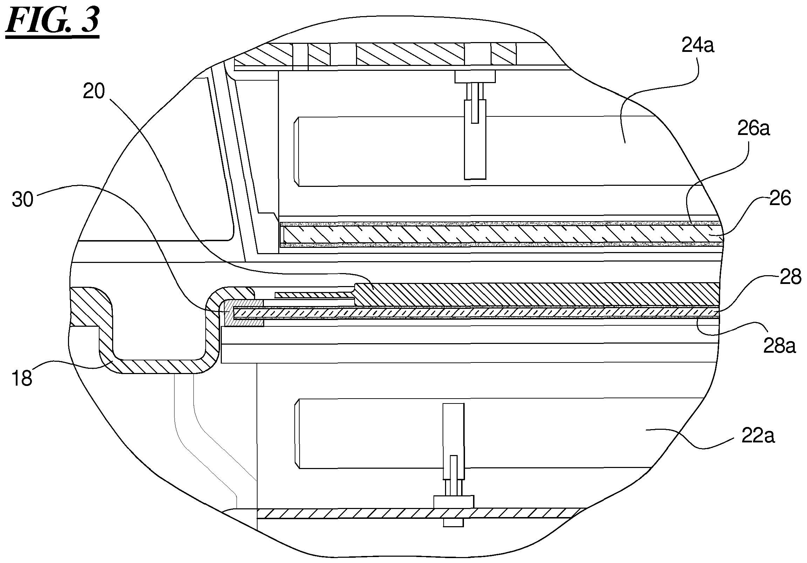

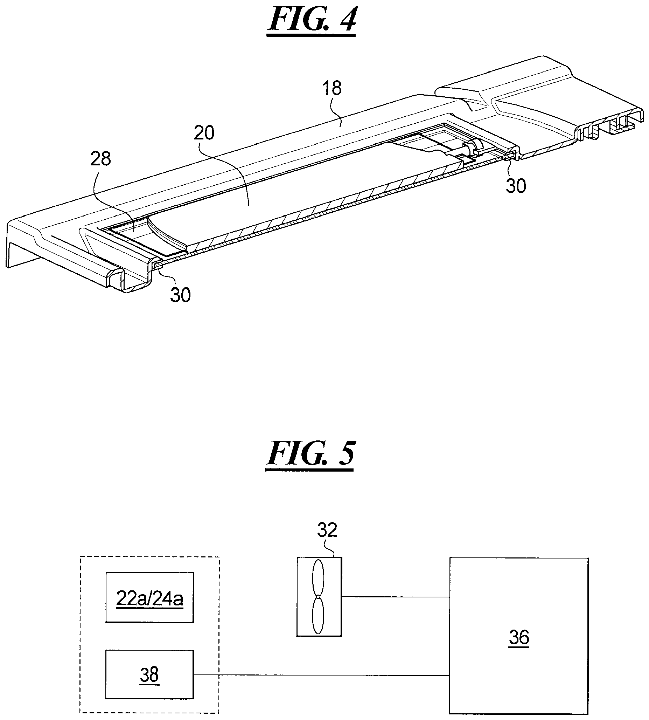

The treatment chamber is defined by a translucent ceiling 26 and a translucent floor 28 that are interposed between the tray 18 and the upper and lower arrays of light sources, 24a, 22a, respectively, to permit the illumination on both sides of the biological fluid container 20. The floor 28 and ceiling 26 may be made of, e.g., glass. As illustrated, the glass sheets forming the floor and ceiling are mounted in a frame 30 (best seen in Figs. 3 and 4) that is attached or secured to, or formed integrally with, the interior of the housing 12, e.g., the base 14 or tray 18 and the cover 16.

The translucent ceiling 26 and floor 28 serve as filters that permit passage of light of the spectrum/wavelength required for treatment of the biological fluid (e.g., activation of a photoactive agent in the biological fluid), but block light outside of the desired spectrum/wavelength, so as to avoid heating of the contents of the treatment container other than that incidental to the absorption of light of the desired spectrum. In the context of the treatment of mononuclear cells, the desired spectrum is UV light having a wavelength in the UVA range of about 320 nm to 400 nm, while the light to be blocked is light falling outside the UVA range, and more specifically, IR light having a wavelength of from about 750 nm to 1 mm.

In keeping with the disclosure, each of the translucent floor 28 and ceiling 26 comprises a first surface facing the tray 18 and a second surface 28a, 26a (best seen in Fig. 3), facing away from the tray 18 and toward its associated array of radiation-emitting bulbs 22a, 24a. The second surface 28a, 26a,of each of the floor 28 and ceiling 26, i.e., that surface facing away from the tray 18, has a coating thereon for blocking and/or reflecting radiation of the selected wavelength. This helps to reflect the undesirable light (e.g., IR) away from the floor and ceiling before being absorbed, and thus prevents warming of the floor and ceiling.

Each of the floor 28 and the ceiling 26 comprises a substrate with a coating thereon for blocking and/or reflecting radiation of the selected wavelength. As noted above the floor and ceiling comprise glass, which provides the substrate and the coating comprises silver. The coating has a thickness of from 0.5 µm to 1.0 µm. The substrate should have a thickness that is not so great as to block UV light, but not so thin as to be unduly prone to breaking. Accordingly, the substrate preferably has a thickness of 3.3 mm ⁺/- 1.3 mm. Glass plates having such characteristics will transmit UV light having a wavelength of 320-400 nm, while blocking IR light having a wave length of 750 nm - 1mm, and may be obtained from, e.g., Shott North America, Inc. of Duryea, PA, under the product designation B-270.

In order to further moderate the temperature of the treatment chamber, the device preferably includes a fan or blower 32 mounted within the base, and the base includes vents 34 to permit air circulation and exchange around the tray. Further, the speed of the fan 32 is preferably controllable to vary the UVA output of the light sources 22a, 24a. Specifically, the radiant output of the light sources varies with temperature, with the radiant output increasing to a maximum as the temperature of the light sources approaches 40°C (at the tube wall) and then decreasing. Thus, the UVA output of the light sources may be increased to or decreased from a maximum value by controlling the temperature of the light sources, which can be accomplished by varying the rate of air flow through the irradiation device 10 by increasing the fan speed (to decrease the temperature of the light sources) or decreasing the fan speed (to increase the temperature of the light sources).

To this end, and with reference to Fig. 5, a UVA sensor 38 is mounted on the interior the compartments for the light sources 22a, 24a in proximity to the light sources. The sensor(s) 38 generate a signal indicative of the radiant output of the light sources that is transmitted to a programmable controller 36. The programmable controller 36 compares the signal from the UVA sensor(s) 38 to a rated maximum radiant output for the light sources, and then controls the speed of the fan 32 to either increase or decrease the ambient temperature of the light sources to adjust the radiant output of the light sources. For example, maximizing the UVA output of the light sources 22a, 24a would shorten the time of irradiation of the fluid container 20.

By way of example, a description of the use of the irradiation device described herein for the treatment of mononuclear cells with ultraviolet light follows. In such a treatment method, whole blood is withdrawn from a patient and introduced into the separation chamber of the cell separator, where the whole blood is subjected to a centrifugal field. The centrifugal field separates the target cell population, i.e., mononuclear cells, from red blood cells, platelets and plasma. The separated red blood cells and platelets may be returned to the patient, or may be diverted to a container for further processing. However, a residual quantity of red blood cells and plasma typically remains in suspension with the separated mononuclear cells.

The suspended mononuclear cells may be combined with a lysing agent and then incubated to activate the lysing agent to disintegrate or dissolve the residual red blood cells. The suspension is then washed with the apheresis device to remove plasma and hemoglobin freed by the lysis of the red blood cells. The washed, lysed suspension is then re-suspended, and combined with an activation agent, and then exposed to ultraviolet light to obtain a treated cell product. In one non-limiting example, during treatment, the mononuclear cell product may be exposed to UV bulbs having a wavelength in the UVA range of about 320 nm to 400 nm for a selected period of time, preferably 5 minutes or less, resulting in an average UVA exposure of approximately 0.5-5.0 J/cm2. Due to the IR reflective/blocking coating on the floor and ceiling that define the treatment chamber, the UVA light needed for treating the mononuclear cell product passes into the treatment chamber, while the undesirable IR light is reflected away, thus avoiding heating of the mononuclear cell product that would have otherwise resulted due to its absorption of the IR light.

The treated cell product is then returned to the patient. Optionally, the treated mononuclear cells may first be returned to separator and concentrated to provide for the concentrated cells to have a smaller total volume as compared to un-concentrated cells. As a result, the smaller volume of concentrated MNCs may be more quickly reinfused to a patient.

## Claims

1. A device (10) for irradiation of a biological product/substance comprising:
a) a base (14);
b) a tray (18) having a first and second side associated with the base (14) and configured to receive a container (20) holding the biological product/substance to be irradiated;
c) a first array (22) of radiation-emitting bulbs (22a) mounted in the device (10) adjacent the first side of the tray (18);
d) a second array (24) of radiation-emitting bulbs (24a) mounted in the device (10) adjacent the second side of the tray (18);
e) a translucent floor as first filter (28) mounted in the device (10) between the tray (18) and the first array (22) of radiation-emitting bulbs (22a) for blocking and/or reflecting radiation emitted by the first array (22) of a selected wavelength; and
f) a translucent ceiling as second filter (26) mounted in the device (10) between the tray (18) and the second array (24) of radiation-emitting bulbs (24a) for blocking and/or reflecting radiation emitted by the second array (24) of the selected wavelength;
g) each of the first and second filters (28, 26) comprising a glass substrate having a first surface facing toward the tray (18) and a second surface (28a, 26a) facing away from the tray (18) and toward its associated array (22, 24) of radiation-emitting bulbs (22a, 24a), the second surface (28a, 26a) of each substrate having a silver coating thereon having a thickness of from 0.5 µm to 1.0 µm for blocking and/or reflecting radiation of the selected wavelength,
h) wherein the first and second filters (28, 26) each blocks and/or reflects infrared (IR) radiation while permitting ultraviolet (UV) radiation to pass therethrough..

2. The device (10) of Claim 1 further comprising the container (20) for holding the biological product/substance to be irradiated and an openable cover (16),
wherein the first array (22) of radiation-emitting bulbs (22a) is mounted in the base (14) beneath the tray (18);
wherein the second array (24) of radiation-emitting bulbs (24a) is mounted in the cover (16);
wherein the first filter (28) is mounted in the base (14) between the tray (18) and the first array (22) of radiation-emitting bulbs (22a) for blocking and/or reflecting radiation emitted by the first array (22) of a selected wavelength; and
wherein the second filter (26) is mounted in the cover (16) between the tray (18) and the second array (24) of radiation-emitting bulbs (24a) for blocking and/or reflecting radiation emitted by the second array (24) of the selected wavelength.

3. The device of claim 1, wherein the substrate has a thickness of from 3.3 mm ⁺/- 1.3 mm.

4. The device of any one of Claims 1-3 further comprising a fan (32) mounted within the base (14), the base (14) including vents (34) to permit air circulation and exchange around the tray (18).

5. The device of Claim 4 further comprising a sensor (38) for measuring the radiant output of the radiation-emitting bulbs (22a, 24a) and a controller (36) configured to adjust the speed of the fan (32) based on a signal received from the sensor (38) to vary the radiant output of the radiation-emitting bulbs (22a, 24a).

6. A method for irradiating a mononuclear cell product comprising:
a) placing a collection container (20) suitable for irradiation into a tray (18) and into an irradiation chamber within a device as defined by any one of claims 1-8 , the irradiation chamber being positioned between first and second light sources (22a, 24a); wherein the irradiation chamber is defined by a translucent ceiling (26) and a translucent floor (28) that serve as first and second filters, wherein each of the translucent ceiling (26) and translucent floor (28) comprises a first surface facing the tray and second surfaces (26a, 28a) facing away from the tray (18), the surfaces (26a, 28a) facing away from the tray being the exterior surface of the chamber having a coating thereon that blocks and/or reflects non UVA light;
b) introducing a suspension comprising mononuclear cells into the collection container (20);
c) activating the first and second light sources (22a, 24a) to introduce UVA light into the irradiation chamber to expose the collection container (20) to UVA light; and
d) preventing non-UVA light from entering into the collection chamber.

## Patentansprüche

1. Vorrichtung (10) zur Bestrahlung eines biologischen Produkts/Substanz, aufweisend:
a) eine Basis (14);
b) eine Schale (18) mit einer ersten und einer zweiten Seite, die der Basis (14) zugeordnet ist und derart konfiguriert ist, dass sie einen Behälter (20) aufnimmt, der das zu bestrahlende biologische Produkt/Substanz enthält;
c) ein erstes Array (22) von Strahlung-emittierenden Lampen (22a), die in der Vorrichtung (10) benachbart der ersten Seite der Schale (18) angebracht sind;
d) ein zweites Array (24) von Strahlung-emittierenden Lampen (24a), die in der Vorrichtung (10) benachbart der zweiten Seite der Schale (18) angebracht sind;
e) einen lichtdurchlässigen Boden als erster Filter (28), der in der Vorrichtung (10) zwischen der Schale (18) und dem ersten Array (22) von Strahlung-emittierenden Lampen (22a) angebracht ist, um die von dem ersten Array (22) emittierte Strahlung einer ausgewählten Wellenlänge zu blockieren und/oder zu reflektieren; und
f) eine lichtdurchlässige Decke als zweiter Filter (26), der in der Vorrichtung (10) zwischen der Schale (18) und dem zweiten Array (24) von Strahlung-emittierenden Lampen (24a) angebracht ist, um die von dem zweiten Array (24) emittierte Strahlung der ausgewählten Wellenlänge zu blockieren und/oder zu reflektieren;
g) wobei jeder der ersten und zweiten Filter (28, 26) ein Glassubstrat mit einer ersten Oberfläche, die der Schale (18) zugewandt ist, und einer zweiten Oberfläche (28a, 26a), die von der Schale (18) weg und hin zu dem ihr zugeordneten Array (22, 24) von Strahlung-emittierenden Lampen (22a, 24a) gewandt ist, aufweist, wobei die zweite Oberfläche (28a, 26a) jedes Substrats eine Silberbeschichtung darauf mit einer Dicke von 0,5 µm bis 1,0 µm aufweist, um Strahlung der ausgewählten Wellenlänge zu blockieren und/oder zu reflektieren,
h) wobei der erste und der zweite Filter (28, 26) jeweils Infrarotstrahlung (IR) blockieren und/oder reflektieren, während sie Ultraviolettstrahlung (UV) durchlassen.

2. Vorrichtung (10) nach Anspruch 1, ferner aufweisend den Behälter (20) zur Aufnahme des zu bestrahlenden biologischen Produkts/Substanz und eine zu öffnende Abdeckung (16), wobei das erste Array (22) von Strahlung-emittierenden Lampen (22a) in der Basis (14) unter der Schale (18) angebracht ist;
wobei das zweite Array (24) von Strahlung-emittierenden Lampen (24a) in der Abdeckung (16) angebracht ist;
wobei der erste Filter (28) in der Basis (14) zwischen der Schale (18) und dem ersten Array (22) von Strahlung-emittierenden Lampen (22a) angebracht ist, um die von dem ersten Array (22) emittierte Strahlung einer ausgewählten Wellenlänge zu blockieren und/oder zu reflektieren; und
wobei der zweite Filter (26) in der Abdeckung (16) zwischen der Schale (18) und dem zweiten Array (24) von Strahlung-emittierenden Lampen (24a) angebracht ist, um die von dem zweiten Array (24) emittierte Strahlung der ausgewählten Wellenlänge zu blockieren und/oder zu reflektieren.

3. Vorrichtung nach Anspruch 1,
wobei das Substrat eine Dicke im Bereich von 3,3 mm +/- 1,3 mm aufweist.

4. Vorrichtung nach einem der Ansprüche 1 - 3,
ferner aufweisend einen Ventilator (32), der innerhalb der Basis (14) angebracht ist, wobei die Basis (14) Entlüftungsöffnungen (34) aufweist, um die Zirkulation und den Austausch von Luft um die Schale (18) herum zu ermöglichen.

5. Vorrichtung nach Anspruch 4,
ferner aufweisend einen Sensor (38) zum Messen der Strahlungsleistung der Strahlung-emittierenden Lampen (22a, 24a) und eine Steuerung (36), die derart konfiguriert ist, dass sie die Drehzahl des Ventilators (32) auf Grundlage eines von dem Sensor (38) empfangenen Signals einstellt, um die Strahlungsleistung der Strahlung-emittierenden Lampen (22a, 24a) zu variieren.

6. Verfahren zur Bestrahlung eines mononuklearen Zellprodukts, aufweisend:
a) Platzieren eines zur Bestrahlung geeigneten Sammelbehälters (20) in eine Schale (18) und in eine Bestrahlungskammer innerhalb einer Vorrichtung, die durch einen der Ansprüche 1 - 8 definiert wird, wobei die Bestrahlungskammer zwischen einer ersten und einer zweiten Lichtquelle (22a, 24a) positioniert ist; wobei die Bestrahlungskammer durch eine lichtdurchlässige Decke (26) und einen lichtdurchlässigen Boden (28) definiert ist, die als erste und zweite Filter dienen, wobei jede der lichtdurchlässigen Decke (26) und des lichtdurchlässigen Bodens (28) eine erste Oberfläche, die der Tischplatte zugewandt ist, und zweite Oberflächen (26a, 28a), die von der Schale (18) abgewandt sind, aufweist, wobei die von der Platte abgewandten Oberflächen (26a, 28a) die Außenfläche der Kammer mit einer Beschichtung darauf sind, die Nicht-UVA-Licht blockiert und/oder reflektiert;
b) Einbringen einer Suspension, die mononukleare Zellen aufweist, in den Sammelbehälter (20);
c) Aktivieren der ersten und zweiten Lichtquellen (22a, 24a), um das UVA-Licht in die Bestrahlungskammer einzuleiten, um den Sammelbehälter (20) dem UVA-Licht auszusetzen; und
d) Verhindern des Eintritts von Nicht-UVA-Licht in die Sammelkammer.

## Revendications

1. Dispositif (10) pour l'irradiation d'un produit/d'une substance biologique comprenant :
a) une base (14) ;
b) un plateau (18) présentant un premier et un deuxième côté associés à la base (14) et configuré pour recevoir un récipient (20) contenant le/la produit/substance biologique à irradier ;
c) un premier arrangement (22) d'ampoules émettrices de rayonnement (22a) monté dans le dispositif (10) de manière adjacente au premier côté du plateau (18) ;
d) un deuxième arrangement (24) d'ampoules émettrices de rayonnement (24a) monté dans le dispositif (10) de manière adjacente au deuxième côté du plateau (18) ;
e) un fond translucide en tant que premier filtre (28) monté dans le dispositif (10) entre le plateau (18) et le premier arrangement (22) d'ampoules émettrices de rayonnement (22a) pour bloquer et/ou réfléchir le rayonnement émis par le premier arrangement (22) d'une longueur d'onde sélectionnée ; et
f) un plafond translucide en tant que deuxième filtre (26) monté dans le dispositif (10) entre le plateau (18) et le deuxième arrangement (24) d'ampoules émettrices de rayonnement (24a) pour bloquer et/ou réfléchir le rayonnement émis par le deuxième arrangement (24) de la longueur d'onde sélectionnée ;
g) chacun des premier et deuxième filtres (28, 26) comprenant un substrat en verre présentant une première surface tournée vers le plateau (18) et une deuxième surface (28a, 26a) tournée à l'opposé du plateau (18) et vers son arrangement associé (22, 24) d'ampoules émettrices de rayonnement (22a, 24a), la deuxième surface (28a, 26a) de chaque substrat présentant un revêtement en argent sur celle-ci avec une épaisseur allant de 0,5 µm à 1,0 µm pour bloquer et/ou réfléchir le rayonnement de la longueur d'onde sélectionnée,
h) dans lequel les premier et deuxième filtres (28, 26) bloquent et/ou réfléchissent chacun un rayonnement infrarouge (IR) tout en permettant au rayonnement ultraviolet (UV) de passer au travers.

2. Dispositif (10) selon la revendication 1, comprenant en outre le récipient (20) pour contenir le/la produit/substance biologique à irradier et un couvercle ouvrable (16), dans lequel le premier arrangement (22) d'ampoules émettrices de rayonnement (22a) est monté dans la base (14) sous le plateau (18) ;
dans lequel le deuxième arrangement (24) d'ampoules émettrices de rayonnement (24a) est monté dans le couvercle (16) ;
dans lequel le premier filtre (28) est monté dans la base (14) entre le plateau (18) et le premier arrangement (22) d'ampoules émettrices de rayonnement (22a) pour bloquer et/ou réfléchir le rayonnement émis par le premier arrangement (22) d'une longueur d'onde sélectionnée ; et
dans lequel le deuxième filtre (26) est monté dans le couvercle (16) entre le plateau (18) et le deuxième arrangement (24) d'ampoules émettrices de rayonnement (24a) pour bloquer et/ou réfléchir le rayonnement émis par le deuxième arrangement (24) de la longueur d'onde sélectionnée.

3. Dispositif selon la revendication 1,
dans lequel le substrat présente une épaisseur allant de 3,3 mm +/- 1,3 mm.

4. Dispositif selon l'une quelconque des revendications 1 - 3,
comprenant en outre un ventilateur (32) monté à l'intérieur de la base (14), la base (14) comportant des évents (34) pour permettre la circulation et l'échange d'air autour du plateau (18).

5. Dispositif selon la revendication 4,
comprenant en outre un capteur (38) pour mesurer la sortie rayonnante des ampoules émettrices de rayonnement (22a, 24a) et un dispositif de commande (36) configuré pour ajuster la vitesse du ventilateur (32) sur la base d'un signal reçu du capteur (38) pour faire varier la sortie rayonnante des ampoules émettrices de rayonnement (22a, 24a).

6. Procédé pour irradier un produit cellulaire mononucléaire comprenant :
a) le placement d'un récipient de collecte (20) approprié pour une irradiation dans un plateau (18) et dans une chambre d'irradiation à l'intérieur d'un dispositif tel que défini dans l'une quelconque des revendications 1 - 8, la chambre d'irradiation étant positionnée entre des première et deuxième sources de lumière (22a, 24a) ; dans lequel la chambre d'irradiation est définie par un plafond translucide (26) et un fond translucide (28) qui font office de premier et deuxième filtres, dans lequel chacun du plafond translucide (26) et du fond translucide (28) comprend une première surface tournée vers le plateau et des deuxièmes surfaces (26a, 28a) tournées à l'opposé du plateau (18), les surfaces (26a, 28a) tournées à l'opposé du plateau étant la surface extérieure de la chambre présentant un revêtement sur celle-ci qui bloque et/ou réfléchit de la lumière non UVA ;
b) l'introduction d'une suspension comprenant des cellules mononucléaires dans le récipient de collecte (20) ;
c) l'activation des première et deuxième sources de lumière (22a, 24a) pour introduire la lumière UVA dans la chambre d'irradiation pour exposer le récipient de collecte (20) à la lumière UVA ; et
d) l'empêchement de la lumière non UVA d'entrer dans la chambre de collecte.
